# EUROPEAN PATENT SPECIFICATION

(11) **EP 4 324 379 B1**
(45) Date of publication and mention of the grant of the patent: **23.09.2026**
(21) Application number: 23191772.5
(22) Date of filing: 16.08.2023
(51) Int. Cl.: A61B 1/00, A61B 1/04

(54) **WIRELESS POWER TRANSFER TO CAPSULE ENDOSCOPE FROM DEVICE USED IN LAPAROSCOPIC SURGERY**
DRAHTLOSE STROMÜBERTRAGUNG AN EIN KAPSELENDOSKOP VON EINER IN DER LAPAROSKOPISCHEN CHIRURGIE VERWENDETEN VORRICHTUNG
TRANSFERT D'ÉNERGIE SANS FIL VERS UN ENDOSCOPE À CAPSULE À PARTIR D'UN DISPOSITIF UTILISÉ EN CHIRURGIE LAPAROSCOPIQUE

(30) Priority: 18.08.2022 US 202263398945 P; 05.07.2023 US 202318346926
(43) Date of publication of application: 21.02.2024
(73) Proprietor: Covidien LP, Mansfield, MA 02048 (US)
(72) Inventor: ZHAO, Jing, Boulder, 80301 (US)
(74) Representative: Zimmermann & Partner Patentanwälte mbB

(56) References cited:
- JP-A- 2001 224 551
- JP-A- 2006 141 725
- US-A1- 2005 154 294
- US-A1- 2021 267 438

## Description

### BACKGROUND

There is a growing interest in deploying a second camera in a minimally invasive surgery to augment the primary view created by a rigid or flexible endoscope inside a patient's abdominal cavity. The main purpose of this effort is to enhance situational awareness and safety during a surgical operation which is often affected by the quality of visualization.

Conventional wirelessly powered devices utilize near-field magnetic inductive coupling and operate in a low frequency band (e.g., about 135 kHz) or high frequency band (e.g., about 13.56 MHz). However, the human body becomes too lossy for higher frequencies to penetrate, thus significantly reducing charging efficiency. This is true if a charging coil is located outside of a patient's body while transferring power to a medical device inside the stomach or small intestine. Also, any metallic or ferromagnetic components in the vicinity alter magnetic field distribution, resulting in suboptimal charging performance.

Document JP2006141725 A discloses an imaging system comprising a direct-view endoscope and a capsule endoscope, wherein the capsule endoscope includes an imaging unit and an antenna configured to transmit images from the imaging unit wirelessly, and wherein the system further includes a camera control unit configured to receive and process image data from both the direct-view endoscope and the capsule endoscope.

### SUMMARY

The present disclosure provides for a medical imaging system including a flexible or rigid endoscope and a capsule endoscope. The capsule endoscope is placed inside a patient in proximity (e.g., up to 20 cm) to other surgical devices (e.g., trocars, straight instruments, endoscope, etc.) in use, and is separated from the other surgical devices by insufflation gas. At least one of the surgical devices includes an antenna, e.g., coil, configured to transmit wireless power to the capsule endoscope. Thus, patient tissue attenuation is not an issue for higher frequency electromagnetic (EM) waves since wireless power is delivered from a surgical device, e.g., the trocar, to the capsule endoscope while both are disposed in the abdominal cavity.

The imaging system includes a radio frequency (RF) power source configured to transmit wireless power at microwave and mmWave frequencies (e.g., ultra-wideband (3.1-10.6 GHz), 5G NR (sub-7 GHz). A transmitter antenna is disposed on one of the medical devices and transmits wireless power to a receiver antenna disposed within the capsule endoscope. Due to the GHz operating frequency, the transmitter antenna is relatively small, e.g., less than 10 cm², and may be integrated into any surgical device. The antenna may be conformal, flexible, and miniaturized allowing for wrapping thereof around a cylindrical object (e.g., a trocar or the shaft of an instrument) with a micro coaxial cable connected to the RF source external to the patient. The power source may be connected to multiple antennas, forming an antenna array, with each of the antennas disposed on one of the multiple instruments thereby maximizing power transfer per unit time. The imaging system also includes a camera control unit configured to communicate wirelessly with the capsule endoscope over a separate wireless spectrum to minimize interference with the power transmission spectrum, which may be 2.45 GHz (e.g., Wi-Fi).

According to one embodiment of the present disclosure, an imaging system is disclosed. The imaging system includes a laparoscopic medical instrument having a distal portion configured to be inserted into a patient and a power transmitting antenna disposed at the distal portion. The imaging system also includes a power source coupled to the power transmitting antenna configured to transmit wireless power. The imaging system also includes a capsule endoscope having a camera configured to capture a first video stream, a first antenna configured to receive the wireless power from the power transmitting antenna, and a second antenna configured to transmit data that may include the first video stream. The system further includes an endoscopic camera configured to capture a second video stream. The system additionally includes a camera control unit configured to receive the first video stream and the second video stream.

Implementations of the above embodiment may include one or more of the following features. According to one aspect of the above embodiment, the power source may be further configured to transmit the wireless power over a first wireless spectrum. The first wireless spectrum may be from about 3 GHz to about 48 GHz, in embodiments may be from about 3 GHz to about 14 GHz and in further embodiments may be from about 24 GHz to about 48GHz to include mmWave to take advantage of 5G wireless technologies. The second antenna may be configured to transmit over a second wireless spectrum that is different from the first wireless spectrum. The camera control unit may be further configured to combine the first video stream and the second video stream. The endoscopic camera may be further configured to transmit the second video stream over the second wireless spectrum. The medical instrument may be an access port.

According to another embodiment of the present disclosure, a capsule endoscope is disclosed. The capsule endoscope includes a camera configured to capture a video stream and a first antenna configured to receive power from a power transmitting antenna disposed inside a patient. The capsule endoscope further includes a second antenna configured to transmit data including the video stream to a camera control unit outside the patient.

Implementations of the above embodiment may include one or more of the following features. According to one aspect of the above embodiment, the first antenna may be configured to receive in a first wireless spectrum. The second antenna may be configured to transmit in a second wireless spectrum. The first wireless spectrum and the second wireless spectrum may be non-overlapping. The first wireless spectrum may be from about 3 GHz to about 14 GHz. The second wireless spectrum may be below 3 GHz.

According to a further embodiment of the present disclosure, a surgical robotic system is disclosed. The surgical robotic system includes a robotic arm having a medical instrument having a power transmitting antenna disposed thereon. The system also includes a control tower having a power source coupled to the power transmitting antenna and configured to transmit wireless power. The system further includes a capsule endoscope having a camera configured to capture a first video stream, a first antenna configured to receive the wireless power from the power transmitting antenna, and a second antenna configured to transmit data including the first video stream. The system additionally includes an endoscopic camera configured to capture a second video stream. The system further includes a camera control unit configured to receive the first video stream and the second video stream.

Implementations of the above embodiment may include one or more of the following features. According to one aspect of the above embodiment, the power source may be further configured to transmit the wireless power over a first wireless spectrum. The first wireless spectrum may be from about 3 GHz to about 14 GHz. The second antenna may be configured to transmit over a second wireless spectrum that is different from the first wireless spectrum. The camera control unit may be further configured to combine the first video stream and the second video stream. The endoscopic camera may be further configured to transmit the second video stream over the second wireless spectrum. The medical instrument may be an access port.

### BRIEF DESCRIPTION OF THE DRAWINGS

Various embodiments of the present disclosure are described herein with reference to the drawings wherein:
FIG. 1 is a schematic diagram of an imaging system according to an embodiment of the present disclosure;
FIG. 2 is perspective, partially-exposed view of the capsule endoscope according to an embodiment of the present disclosure;
FIG. 3 is a schematic view of a wireless power transfer system for the capsule endoscope according to an embodiment of the present disclosure; and
FIG. 4 is a schematic illustration of a surgical robotic system including a control tower, a console, and one or more surgical robotic arms each disposed on a mobile cart including the imaging system according to an embodiment of the present disclosure.

### DETAILED DESCRIPTION

Embodiments of the presently disclosed imaging systems are described in detail with reference to the drawings, in which like reference numerals designate identical or corresponding elements in each of the several views.

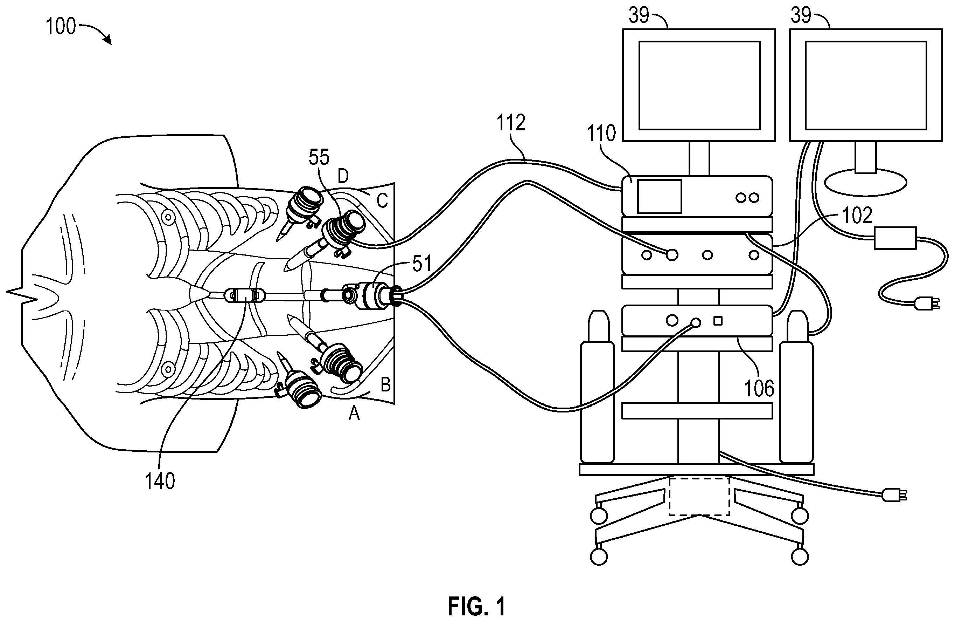

With reference to FIG. 1, an imaging system 100 includes a camera control unit (CCU) 102 configured to couple to one or more cameras, such as an endoscopic camera 51. The system 100 also includes a light source 106 coupled to the camera 51. The light source 106 may include any suitable light sources, e.g., white light, near infrared, etc., having light emitting diodes, lamps, laser, etc. The system 100 also includes a power source 110 configured to transmit RF power in an ultra-wideband power spectrum from about 3 GHz to about 48 GHz. The power source 110 is coupled to one or more antennas 130, each of which is disposed on one of the medical instruments, which may include trocars, access ports 55, graspers, vessel sealers, etc. The imaging system 100 also includes a capsule endoscope 140 that is placed inside the patient. This may be done by dropping the camera into the patient through one of the access ports 55 and maneuvering the capsule endoscope 140 until a desired field of view is achieved. The position of the capsule endoscope 140 may be adjusted throughout the procedure. After the procedure is completed, the capsule endoscope 140 may be picked up by a grasper, a magnet, or any other suitable instrument.

The CCU 102 is configured to receive image data signals, process the raw image data from the camera 51 and the capsule endoscope 140, and may generate blended white light, NIR images for recording and/or real-time display. The CCU 102 is also configured to blend images using various AI image augmentations.

The CCU 102 is connected to the camera 51 using a wired or a wireless interface, which uses a second wireless spectrum for transmitting data, to avoid interference with the power transmission spectrum. The capsule endoscope 140 communicates with the CCU 102 using the wireless interface. The CCU 102 receives image data from the camera 51 and the capsule endoscope 140 and provides the video data to one or more processing units, which are configured to perform operations, calculations, and/or sets of instructions described in the disclosure, and may be a hardware processor, a field programmable gate array (FPGA), a digital signal processor (DSP), a central processing unit (CPU), a microprocessor, and combinations thereof. Those skilled in the art will appreciate that the processor may be any logic processor (e.g., control circuit) adapted to execute algorithms, calculations, and/or sets of instructions as described herein. The processing unit may also be a graphics processing unit (GPU) or an FPGA, which is capable of more parallel executions than a CPU due to a larger number of cores, e.g., thousands of compute unified device architecture (CUDA) cores, making it more suitable for processing images.

The CCU 102 also includes various other computer components, such as RAM, a storage drive, peripheral ports, input device (e.g., touch screen). Additionally, the CCU 102 is also coupled to one or more monitors 39 via output ports. The CCU 102 is configured to output the processed images from the camera 51 and the capsule endoscope 140 through any suitable video output port, such as a DISPLAYPORT^{™}, HDMI^{®}, SDI, etc., that is capable of transmitting processed images at any desired resolution, display rate, and/or bandwidth.

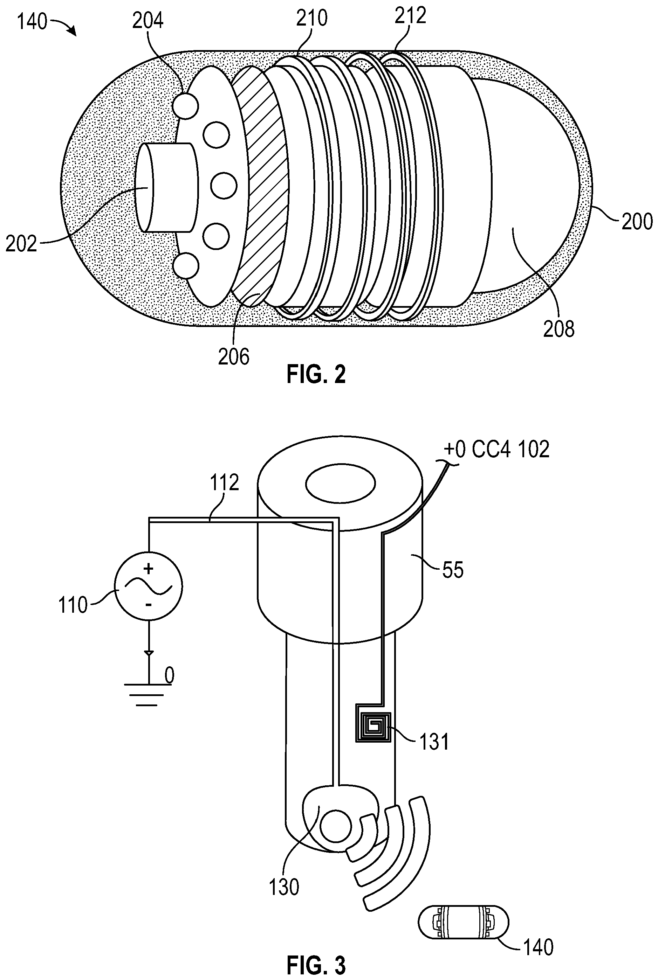

With reference to FIG. 2, the capsule endoscope 140 includes a housing 200 having a camera 202, one or more lights (e.g., LEDs) 204, controller 206, a battery 208, a power antenna 210, and a data antenna 212. The housing 200 may be made of a dielectric material, e.g., one or more thermoplastic polymers, or other non-lossy material that is safe to ingest and seals the components of the endoscope 140 from the outside environment. Lossy materials include metals and other materials which may increase losses in the oscillating magnetic field.

FIG. 3 shows the power source 110 configured to wirelessly transmit power to the capsule endoscope 140. The power source 110 may include an RF power supply coupled via a transmission line 112, e.g., micro coaxial cable, to the antenna 130 that is disposed on the instrument 50, which is shown as an access port 55. The antenna 130 be conformal, flexible, and miniaturized allowing for wrapping thereof around a longitudinal shaft of the access port 55 or any other portion thereof or that of any other instrument 50. In embodiments, the power source 110 may be coupled to a plurality of antennas 130, each outputting an emission field that may overlap with each other to provide uninterrupted power to the capsule endoscope 140. The antennas 130 are disposed inside the patient along with the capsule endoscope 140 power thereby avoiding patient tissue attenuation.

The capsule endoscope 140 receives the wireless power transmission at the power antenna 210 and converts the wireless transmission into electricity to charge the battery 208 and/or power other components, e.g., camera 202, directly. Image capture through the camera 51 is received at the controller 206 and is then transmitted through the data antenna 212, which communicates with the CCU 102 over the data wireless spectrum, e.g., below 3 GHz. The data wireless spectrum does not overlap with the power spectrum to avoid interference. The CCU 102 may also be coupled to one or more data transmissions antennas 131 disposed on the access port 55. In embodiments, the antennas may be disposed outside the patient as the signals in the data wireless spectrum are less susceptible to attenuation by tissue. After receiving the image data wirelessly from the capsule endoscope 140, the CCU 102 then processes the raw image data from the capsule endoscope 140 and the camera 51 for display on the monitors 39. The CCU 102 may combine the image streams, e.g., stitch, overlap, etc., or otherwise augment the video stream received from the camera 51. This allows a narrow field of view of the endoscopic camera 51 to be supplemented with the video stream from the capsule endoscope 140 providing a wider view of the surgical site during laparoscopic surgery. The combined view of the two video streams may be displayed one or more of the monitors 39.

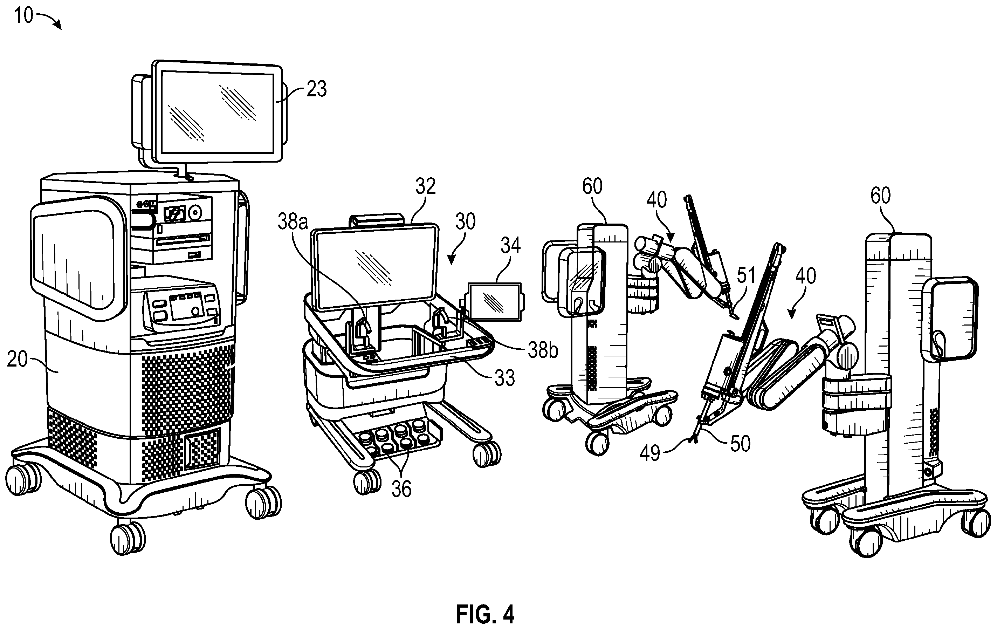

The imaging system 100 may also be incorporated into a surgical robotic system 10, as shown in FIG. 4. The system 10, which includes a control tower 20, which is connected to all of the components of the surgical robotic system 10 including a surgeon console 30 and one or more movable carts 60. The CCU 102 and the power source 110 may be disposed in the control tower 20.

Each of the movable carts 60 includes a robotic arm 40 having a surgical instrument 50 removably coupled thereto. The robotic arms 40 also couple to the movable carts 60. The robotic system 10 may include any number of movable carts 60 and/or robotic arms 40.

The surgical instrument 50 is configured for use during minimally invasive surgical procedures. In embodiments, the surgical instrument 50 may be an endoscope, such as the endoscopic camera 51, configured to provide a video feed for the user. In further embodiments, the surgical instrument 50 may be an electrosurgical forceps configured to seal tissue by compressing tissue between jaw members and applying electrosurgical current thereto. In yet further embodiments, the surgical instrument 50 may be a surgical stapler including a pair of jaws configured to grasp and clamp tissue while deploying a plurality of tissue fasteners, e.g., staples, and cutting stapled tissue.

One of the robotic arms 40 may include the endoscopic camera 51 configured to capture video of the surgical site. The endoscopic camera 51 may be a stereoscopic endoscope configured to capture two side-by-side (i.e., left and right) images of the surgical site to produce a video stream of the surgical scene.

The surgeon console 30 includes a first display 32, which displays a video feed of the surgical site provided by camera 51 and/or the capsule endoscope 140. The surgeon console 30 also includes a second display 34, which displays a user interface for controlling the surgical robotic system 10. The first and second displays 32 and 34 are touchscreens allowing for displaying various graphical user inputs.

The surgeon console 30 also includes a plurality of user interface devices, such as foot pedals 36 and a pair of handle controllers 38a and 38b which are used by a user to remotely control robotic arms 40. The surgeon console further includes an armrest 33 used to support clinician's arms while operating the handle controllers 38a and 38b.

The control tower 20 also includes a display 23, which may be a touchscreen, and outputs on the graphical user interfaces (GUIs). The control tower 20 also acts as an interface between the surgeon console 30 and one or more robotic arms 40. In particular, the control tower 20 is configured to control the robotic arms 40, such as to move the robotic arms 40 and the corresponding surgical instrument 50, based on a set of programmable instructions and/or input commands from the surgeon console 30, in such a way that robotic arms 40 and the surgical instrument 50 execute a desired movement sequence in response to input from the foot pedals 36 and the handle controllers 38a and 38b.

During use, the surgical instrument 50 or any other laparoscopic device (e.g., access port 55, camera 51, etc.) having the antenna 130 and optionally the antenna 131 disposed thereon is inserted into the patient. In addition, the capsule endoscope 140 is also inserted into the patient through the access port 55 or any other opening. Once in place, the capsule endoscope 140 is energized and power and data transmission are established through the antennae 130 and 131, respectively. Power is supplied by the power source 110 and video data is received by the CCU 102, as described above.

It will be understood that various modifications may be made to the embodiments disclosed herein. Therefore, the above description should not be construed as limiting, but merely as exemplifications of various embodiments. Those skilled in the art will envision other modifications within the scope of the invention as defined by the claims appended thereto.

## Claims

1. An imaging system (100) comprising:
a laparoscopic medical instrument (50, 55) including a distal portion configured to be inserted into a patient and a power transmitting antenna (130, 131) disposed at the distal portion;
a power source (110) coupled to the power transmitting antenna configured to transmit wireless power;
a capsule endoscope (140) including:
a camera (202) configured to capture a first video stream;
a first antenna (210) configured to receive the wireless power from the power transmitting antenna when the power transmitting antenna is disposed inside the patient; and
a second antenna (212) configured to transmit data including the first video stream;
an endoscopic camera (51) configured to capture a second video stream; and
a camera control unit (102) configured to receive the first video stream and the second video stream.

2. The imaging system according to claim 1, wherein the power source is further configured to transmit the wireless power over a first wireless spectrum., optionally wherein the first wireless spectrum is from about 3 GHz to about 14 GHz.

3. The imaging system according to claim 2, wherein the second antenna is configured to transmit over a second wireless spectrum that is different from the first wireless spectrum, optionally wherein the endoscopic camera is further configured to transmit the second video stream over the second wireless spectrum.

4. The imaging system according to any preceding claim, wherein the camera control unit is further configured to combine the first video stream and the second video stream.

5. The imaging system according to any preceding claim, wherein the medical instrument is an access port (55).

6. A surgical robotic system (10) comprising:
a robotic arm (40) including a medical instrument (50, 55) having a power transmitting antenna (130, 131) disposed thereon;
a control tower (60) including a power source (110) coupled to the power transmitting antenna and configured to transmit wireless power;
a capsule endoscope (140) including:
a camera (202) configured to capture a first video stream;
a first antenna (210) configured to receive the wireless power from the power transmitting antenna when the power transmitting antenna is disposed inside the patient; and
a second antenna (212) configured to transmit data including the first video stream;
an endoscopic camera (51) configured to capture a second video stream; and
a camera control unit (102) configured to receive the first video stream and the second video stream.

7. The surgical robotic system according to claim 6, wherein the power source is configured to transmit the wireless power over a first wireless spectrum.

8. The surgical robotic system according to claim 7, wherein the first wireless spectrum is from about 3 GHz to about 14 GHz.

9. The surgical robotic system according to claim 7 or 8, wherein the second antenna is configured to transmit over a second wireless spectrum that is different from the first wireless spectrum.

10. The surgical robotic system according to claim 9, wherein the endoscopic camera is configured to transmit the second video stream over the second wireless spectrum.

11. The surgical robotic system according to any one of Claims 6 to 10, wherein the camera control unit is configured to combine the first video stream and the second video stream.

12. The surgical robotic system according to any one of claims 6 to 11, wherein the medical instrument is an access port (55).

## Patentansprüche

1. Bildgebungssystem (100), umfassend:
ein laparoskopisches medizinisches Instrument (50, 55), einschließlich einem distalen Abschnitt, der zum Einführen in einen Patienten konfiguriert ist, und einer Energieübertragungsantenne (130, 131), die an dem distalen Abschnitt angeordnet ist;
eine Energiequelle (110), die mit der Energieübertragungsantenne gekoppelt ist, die dazu konfiguriert ist, drahtlose Energie zu übertragen;
ein Kapselendoskop (140), einschließlich:
einer Kamera (202), die konfiguriert ist, einen ersten Video-Stream aufzunehmen;
einer ersten Antenne (210), die konfiguriert ist, die drahtlose Energie von der Energieübertragungsantenne zu empfangen, wenn die Energieübertragungsantenne im Inneren des Patienten angeordnet ist; und
einer zweiten Antenne (212), die konfiguriert ist, Daten zu übertragen, die den ersten Video-Stream einschließen;
eine endoskopische Kamera (51), die konfiguriert ist, einen zweiten Video-Stream aufzunehmen; und
eine Kamerasteuereinheit (102), die konfiguriert ist, den ersten Video-Stream und den zweiten Video-Stream zu empfangen.

2. Bildgebungssystem nach Anspruch 1, wobei die Energiequelle ferner konfiguriert ist, die drahtlose Energie über ein erstes drahtloses Spektrum zu übertragen, wobei optional das erste drahtlose Spektrum von etwa 3 GHz bis etwa 14 GHz reicht.

3. Bildgebungssystem nach Anspruch 2, wobei die zweite Antenne konfiguriert ist, über ein zweites drahtloses Spektrum zu übertragen, das sich von dem ersten drahtlosen Spektrum unterscheidet, wobei optional die endoskopische Kamera ferner konfiguriert ist, den zweiten Video-Stream über das zweite drahtlose Spektrum zu übertragen.

4. Bildgebungssystem nach einem der vorstehenden Ansprüche, wobei die Kamerasteuereinheit ferner konfiguriert ist, den ersten Video-Stream und den zweiten Video-Stream zu kombinieren.

5. Bildgebungssystem nach einem der vorstehenden Ansprüche, wobei das medizinische Instrument ein Zugriffsport (55) ist.

6. Chirurgisches Robotersystem (10), umfassend:
einen Roboterarm (40), der ein medizinisches Instrument (50, 55) einschließt, das eine daran angeordnete Energieübertragungsantenne (130, 131) aufweist;
einen Steuerturm (60), der eine Energiequelle (110) einschließt, die mit der Energieübertragungsantenne gekoppelt und konfiguriert ist, drahtlose Energie zu übertragen;
ein Kapselendoskop (140), einschließlich:
einer Kamera (202), die konfiguriert ist, einen ersten Video-Stream aufzunehmen;
einer ersten Antenne (210), die konfiguriert ist, die drahtlose Energie von der Energieübertragungsantenne zu empfangen, wenn die Energieübertragungsantenne im Inneren des Patienten angeordnet ist; und
einer zweiten Antenne (212), die konfiguriert ist, Daten zu übertragen, die den ersten Video-Stream einschließen;
eine endoskopische Kamera (51), die konfiguriert ist, einen zweiten Video-Stream aufzunehmen; und
eine Kamerasteuereinheit (102), die konfiguriert ist, den ersten Video-Stream und den zweiten Video-Stream zu empfangen.

7. Chirurgisches Robotersystem nach Anspruch 6, wobei die Energiequelle konfiguriert ist, die drahtlose Energie über ein erstes drahtloses Spektrum zu übertragen.

8. Chirurgisches Robotersystem nach Anspruch 7, wobei das erste drahtlose Spektrum von etwa 3 GHz bis etwa 14 GHz reicht.

9. Chirurgisches Robotersystem nach Anspruch 7 oder 8, wobei die zweite Antenne konfiguriert ist, über ein zweites drahtloses Spektrum zu übertragen, das sich von dem ersten drahtlosen Spektrum unterscheidet.

10. Chirurgisches Robotersystem nach Anspruch 9, wobei die endoskopische Kamera konfiguriert ist, den zweiten Video-Stream über das zweite drahtlose Spektrum zu übertragen.

11. Chirurgisches Robotersystem nach einem der Ansprüche 6 bis 10, wobei die Kamerasteuereinheit konfiguriert ist, den ersten Video-Stream und den zweiten Video-Stream zu kombinieren.

12. Chirurgisches Robotersystem nach einem der Ansprüche 6 bis 11, wobei das medizinische Instrument ein Zugriffsport (55) ist.

## Revendications

1. Système d'imagerie (100), comprenant :
un instrument médical laparoscopique (50, 55) comportant une partie distale conçue pour être insérée dans un patient et une antenne d'émission d'énergie (130, 131) disposée au niveau de la partie distale ;
une source d'énergie (110) couplée à l'antenne d'émission d'énergie configurée pour transmettre de l'énergie sans fil ;
une capsule endoscopique (140) comportant :
une caméra (202) configurée pour capturer un premier flux vidéo ;
une première antenne (210) configurée pour recevoir l'énergie sans fil depuis l'antenne d'émission d'énergie lorsque l'antenne d'émission d'énergie est disposée à l'intérieur du patient ; et
une seconde antenne (212) configurée pour transmettre des données comportant le premier flux vidéo ; une caméra endoscopique (51) configurée pour capturer un second flux vidéo ; et
une unité de commande de caméra (102) configurée pour recevoir le premier flux vidéo et le second flux vidéo.

2. Système d'imagerie selon la revendication 1, dans lequel la source d'énergie est en outre configurée pour transmettre l'énergie sans fil sur un premier spectre sans fil, éventuellement dans lequel le premier spectre sans fil va d'environ 3 GHz à environ 14 GHz.

3. Système d'imagerie selon la revendication 2, dans lequel la seconde antenne est configurée pour transmettre sur un second spectre sans fil qui est différent du premier spectre sans fil, éventuellement dans lequel la caméra endoscopique est en outre configurée pour transmettre le second flux vidéo sur le second spectre sans fil.

4. Système d'imagerie selon l'une quelconque revendication précédente, dans lequel l'unité de commande de caméra est en outre configurée pour combiner le premier flux vidéo et le second flux vidéo.

5. Système d'imagerie selon l'une quelconque revendication précédente, dans lequel l'instrument médical est un port d'accès (55).

6. Système robotique chirurgical (10) comprenant :
un bras robotique (40) comportant un instrument médical (50, 55) ayant une antenne d'émission d'énergie (130, 131) disposée sur celui-ci ;
une tour de commande (60) comportant une source d'énergie (110) couplée à l'antenne d'émission d'énergie et configurée pour transmettre de l'énergie sans fil ;
une capsule endoscopique (140) comportant :
une caméra (202) configurée pour capturer un premier flux vidéo ;
une première antenne (210) configurée pour recevoir l'énergie sans fil depuis l'antenne d'émission d'énergie lorsque l'antenne d'émission d'énergie est disposée à l'intérieur du patient ; et
une seconde antenne (212) configurée pour transmettre des données comportant le premier flux vidéo ; une caméra endoscopique (51) configurée pour capturer un second flux vidéo ; et
une unité de commande de caméra (102) configurée pour recevoir le premier flux vidéo et le second flux vidéo.

7. Système robotique chirurgical selon la revendication 6, dans lequel la source d'énergie est configurée pour transmettre l'énergie sans fil sur un premier spectre sans fil.

8. Système robotique chirurgical selon la revendication 7, dans lequel le premier spectre sans fil va d'environ 3 GHz à environ 14 GHz.

9. Système robotique chirurgical selon la revendication 7 ou 8, dans lequel la seconde antenne est configurée pour transmettre sur un second spectre sans fil qui est différent du premier spectre sans fil.

10. Système robotique chirurgical selon la revendication 9, dans lequel la caméra endoscopique est configurée pour transmettre le second flux vidéo sur le second spectre sans fil.

11. Système robotique chirurgical selon l'une quelconque des revendications 6 à 10, dans lequel l'unité de commande de caméra est configurée pour combiner le premier flux vidéo et le second flux vidéo.

12. Système robotique chirurgical selon l'une quelconque des revendications 6 à 11, dans lequel l'instrument médical est un port d'accès (55).
